# EUROPEAN PATENT SPECIFICATION

(11) **EP 2 146 686 B1**
(45) Date of publication and mention of the grant of the patent: **24.07.2013**
(21) Application number: 08751302.4
(22) Date of filing: 22.05.2008
(51) Int. Cl.: A61K 8/891, A61K 8/898, A61K 8/895, A61Q 5/12

(54) **Hair conditioning composition comprising polyalkylsiloxane mixture, aminosilicone and silicone copolymer emulsion**
Haarpflegezusammensetzung mit einer Polyalkylsiloxan-Mischung, Aminosilikon und einer Silikon-Copolymer-Emulsion
Composition d'après-shampooing comprenant un mélange de polyalkylsiloxane, un aminosilicone et une émulsion de copolymère de silicone

(30) Priority: 23.05.2007 US 931461 P
(43) Date of publication of application: 27.01.2010
(73) Proprietor: The Procter & Gamble Company, Cincinnati, OH 45202 (US)
(72) Inventor: UEHARA, Nobuaki, Hyogo 658-0082 (JP)
(74) Representative: Holmes, Rosalind
(86) International application number: PCT/IB2008/052024
(87) International publication number: WO 2008/142658

(56) References cited:
- WO-A-03/047544
- WO-A-2006/138201
- US-A1- 2006 057 095

## Description

### FIELD OF THE INVENTION

The present invention relates to a hair conditioning composition comprising: a polyalkylsiloxane mixture, an aminosilicone, and a silicone copolymer emulsion. The composition of the present invention provide clean feel and/or reduced tackiness, while providing such improved conditioning benefits such as smooth feel and reduced friction.

### BACKGROUND OF THE INVENTION

Human hair becomes soiled due to its contact with the surrounding environment and from the sebum secreted by the scalp. The soiling of hair causes it to have a dirty feel and an unattractive appearance. The soiling of the hair necessitates shampooing with frequent regularity.

Shampooing cleans the hair by removing excess soil and sebum. However, shampooing can leave the hair in a wet, tangled, and generally unmanageable state. Once the hair dries, it is often left in a dry, rough, lusterless, or frizzy condition due to removal of the hair's natural oils and other natural conditioning and moisturizing components. The hair can further be left with increased levels of static upon drying, which can interfere with combing and result in a condition commonly referred to as "fly-away hair," or contribute to an undesirable phenomenon of "split ends." Further, chemical treatments, such as perming, bleaching, or coloring hair, can also damage hair and leave it dry, rough, lusterless, and damaged.

A variety of approaches have been developed to condition the hair. A common method of providing conditioning benefits to the hair is through the use of conditioning agents such as cationic surfactants and polymers, high melting point fatty compounds, low melting point oils, silicone compounds, and mixtures thereof.

However, there still exists a desire for hair conditioning compositions which provide improved conditioning benefits such as smooth feel and reduced friction. A variety of approaches have been developed to obtain such conditioning benefits. For example, PCT publication WO2006/138201 discloses hair conditioning composition comprising a silicone polymer containing quaternary groups and a gel matrix in Claim 1. PCT publication WO2006/138201 also discloses hair conditioning compositions further containing 2.0% HMW2220 silicone emulsion and 0.8% Aminosilicone in Examples 14 and 18.

The PCT publication WO 2003/047544 discloses shampoo compositions comprising a silicone in water emulsion and further conditioning silicone ingredients.

However, there remains a need for hair conditioning compositions which provide clean feel and/or reduced tackiness, while providing such improved conditioning benefits such as smooth feel and reduced friction.

None of the existing art provides all of the advantages and benefits of the present invention.

### SUMMARY OF THE INVENTION

The present invention is directed to a hair conditioning composition comprising by weight:
(a) from 0.5% 10% of a polyalkylsiloxane mixture comprising:
   (i) a first polyalkylsiloxane which is non-volatile, contains 0% of amino groups, and has a viscosity of from 10,000,000 mm²· s⁻¹ to 20,000,000 mm²· s⁻¹; and
   (ii) a second polyalkylsiloxane which is non-volatile, contains 0% amino groups, and has a viscosity of from 20 mm²· s⁻¹ to 350 mm²· s⁻¹;
(b) from 0.1% to 5% of an aminosilicone having less than about 0.5% nitrogen by weight of the aminosilicone;
(c) from 0.1% to 5% of a silicone copolymer emulsion with an internal phase viscosity of greater than about 100 x 10⁶ mm²/s, as measured at 25°C; and
(d) an aqueous carrier,
wherein the composition contains 1% or less of anionic surfactants and anionic polymers; and wherein the weight ratio of the first polysiloxane to the second polysiloxane is from 1:2 to 1:9.

The conditioning compositions of the present invention provide clean feel and/or reduced tackiness, while providing improved conditioning benefits such as smooth feel and reduced friction.

These and other features, aspects, and advantages of the present invention will become better understood from a reading of the following description, and appended claims.

### DETAILED DESCRIPTION OF THE INVENTION

While the specification concludes with claims particularly pointing out and distinctly claiming the invention, it is believed that the present invention will be better understood from the following description.

Herein, "comprising" means that other steps and other ingredients which do not affect the end result can be added. This term encompasses the terms "consisting of" and "consisting essentially of".

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Herein, "mixtures" is meant to include a simple combination of materials and any compounds that may result from their combination.

### COMPOSITION

It is believed that; the combination of the polyalkylsiloxane mixture, the aminosilicone, and the silicone copolymer emulsion provides balanced benefits between clean feel and improved conditioning.

In the composition of the present invention, it is preferred to use the aminosilicone at a level such that the weight ratio of the polyalkylsiloxane mixture to the aminosilicone is from 1:1 to 20:1, more preferably 4:1 to 16:1, still more preferably 6:1 to 10:1, in view of delivering the balanced benefits, i.e., balance between "clean feel and/or reduced tackiness" and "improved conditioning benefits such as smooth feel and reduced friction".

It is also preferred to contain the polyalkylsiloxane mixture, aminosilicone and silicone copolymer emulsion are contained at a level such that the weight ratio of (i) a total of amount of the polyalkylsiloxane mixture and aminosilicone to (ii) the silicone copolymer emulsion is from about 1:4 to about 10:1, more preferably from about 1:2 to 7.5:1, still more preferably from about 1:1 to about 5:1, in view of delivering the balanced benefits.

Preferably, when containing cationic surfactants and/or gel matrix formed by cationic surfactants and high melting point fatty compounds, the composition of the present invention is substantially free of anionic surfactants and anionic polymers, in view of avoiding undesirable interaction with cationic surfactant and/or in view of stability of the gel matrix. In the present invention, "substantially free of anionic surfactants and anionic polymers" means that the composition contains 1% or less, preferably 0.5% or less, more preferably totally 0% of total of anionic surfactants and anionic polymers.

### POLYALKYLSILOXANE MIXTURE

The compositions of the present invention comprise a polyalkylsiloxane mixture, which comprises: (i) a first polyalkylsiloxane which is non-volatile, contains 0% of amino groups, and has a viscosity of from 10,000,000 mm²· s⁻¹ to 20,000,000 mm²· s⁻¹; (ii) a second polyalkylsiloxane which is non-volatile, contains 0% of amino groups, and has a viscosity of from 20 mm²· s⁻¹ to 350 mm²· s⁻¹. It is believed that; this specific mixture, when used together with the aminosilicone and the silicone copolymer emulsion, can provide better friction reduction and improved clean feel and/or reduced tackiness, compared to other polysiloxanes. Such other polysiloxanes are, for example, the first polyalkylsiloxane per se, the second polyalkylsiloxane per se, and a mixture of the first polyalkylsiloxane with volatile solvents such as cyclomethicone and isoparaffin.

Preferably, the polysiloxane mixture has a viscosity of from about 500 mm²· s⁻¹ to about 100,000 mm²· s⁻¹, more preferably from about 1,000 mm²· s⁻¹ to about 50,000 mm². s⁻¹, still more preferably from about 2,000 mm²· s⁻¹ to about 30,000 mm²· s⁻¹, even more preferably from about 5,000 mm²· s⁻¹ to about 20,000 mm²· s⁻¹. The viscosity can be measured by means of, for example, a glass capillary viscometer as set forth in Dow Coming Corporate Test Method CTM0004, July 20, 1970.

The first and the second polysiloxanes are contained at a level such that the weight ratio of the first polysiloxane to the second polysiloxane is from 1:2 to 1:9, preferably from 1:4 to 1:6, in view of delivering better friction reduction and improved clean feel and/or reduced tackiness.

The polyalkylsiloxane mixture is used at a level by weight of the composition of from 0.5% to 10%, more preferably from 2% to 8%, still more preferably 3% to 5% in view of providing reduced friction.

### FIRST POLYALKYLSILOXANE

The hair conditioning composition of the present invention comprises a first polysiloxane. The first polysiloxane is non-volatile, and contains 0% of amino groups. In the present invention, the first polysiloxanes being "substantially free of amino groups" means that the first polysiloxane contains 0 % of amino groups. The first polysiloxane has a viscosity of from 10,000,000 mm²· s⁻¹ to 20,000,000 mm²· s⁻¹ at 25°C. The first polysiloxane has a molecular weight of preferably from about 100,000 to about 1,000,000, more preferably from about 130,000 to about 800,000, still more preferably from about 230,000 to about 600,000. The first polysiloxane is preferably nonionic.

Preferred first non-volatile polysiloxane useful herein include those with the following structure: 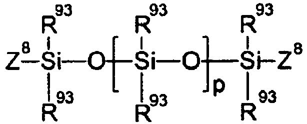 wherein R⁹³ is alkyl or aryl, and p is an integer from about 1,300 to about 15,000, more preferably from about 1,700 to about 11,000, still more preferably from about 3,000 to about 8,000. Z⁸ represents groups which block the ends of the silicone chains. The alkyl or aryl groups substituted on the siloxane chain (R⁹³) or at the ends of the siloxane chains Z⁸ can have any structure as long as the resulting silicone remains fluid at room temperature, is dispersible, is neither irritating, toxic nor otherwise harmful when applied to the hair, is compatible with the other components of the composition, is chemically stable under normal use and storage conditions, and is capable of being deposited on and conditions the hair. Suitable Z⁸ groups include hydroxy, methyl, methoxy, ethoxy, propoxy, and aryloxy. The two R⁹³ groups on the silicon atom may represent the same group or different groups. Preferably, the two R⁹³ groups represent the same group. Suitable R⁹³ groups include methyl, ethyl, propyl, phenyl, methylphenyl and phenylmethyl. The preferred silicone compounds are polydimethylsiloxane, polydiethylsiloxane, and polymethylphenylsiloxane. Polydimethylsiloxane, which is also known as dimethicone, is especially preferred. Commercially available these silicone compounds useful herein include, for example, those available from the General Electric Company in their TSF451 series, and those available from Dow Coming in their Dow Coming SH200 series.

The silicone compounds that can be used herein also include a silicone gum. The term "silicone gum", as used herein, means a polyorganosiloxane material having a viscosity at 25°C of greater than or equal to 1,000,000 mm²· s⁻¹. It is recognized that the silicone gums described herein can also have some overlap with the above-disclosed silicone compounds. This overlap is not intended as a limitation on any of these materials. The "silicone gums" will typically have a mass molecular weight in excess of about 165,000, generally between about 165,000 and about 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof. Commercially available silicone gums useful herein include, for example, TSE200A available from the General Electric Company.

### SECOND POLYALKYLSILOXANE

The hair conditioning composition of the present invention comprises a second polysiloxane. The second polysiloxane is non-volatile, and contains 0% of amino groups. In the present invention, the second polysiloxane being "substantially free of amino groups" means that the second polysiloxane contains 0% of amino groups. The second polysiloxane has a viscosity of from 20 mm²· s⁻¹ to 350 mm²· s⁻¹ at 25°C. The second polysiloxane has a molecular weight of preferably from about 400 to about 65,000, more preferably from about 800 to about 50,000, still more preferably from about 400 to about 30,000, even more preferably from about 400, to about 15,000. The second polysiloxane is preferably nonionic. The second polysiloxane is preferably a linear silicone.

Preferred second non-volatile silicone compounds useful herein include polyalkyl or polyaryl siloxanes with the following structure: 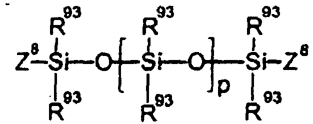 wherein R⁹³ is alkyl or aryl, and p is an integer from about 7 to about 850, more preferably from about 7 to about 665, still more preferably from about 7 to about 400, even more preferred from about 7 to about 200. Z⁸ represents groups which block the ends of the silicone chains. The alkyl or aryl groups substituted on the siloxane chain (R⁹³) or at the ends of the siloxane chains Z⁸ can have any structure as long as the resulting silicone remains fluid at room temperature, is dispersible, is neither irritating, toxic nor otherwise harmful when applied to the hair, is compatible with the other components of the composition, is chemically stable under normal use and storage conditions, and is capable of being deposited on and conditions the hair. Suitable Z⁸ groups include hydroxy, methyl, methoxy, ethoxy, propoxy, and aryloxy. The two R⁹³ groups on the silicon atom may represent the same group or different groups. Preferably, the two R⁹³ groups represent the same group. Suitable R⁹³ groups include methyl, ethyl, propyl, phenyl, methylphenyl and phenylmethyl. The preferred silicone compounds are polydimethylsiloxane, polydiethylsiloxane, and polymethylphenylsiloxane. Polydimethylsiloxane, which is also known as dimethicone, is especially preferred. Commercially available these silicone compounds useful herein include, for example, those available from the General Electric Company in their TSF451 series, and those-available from Dow Corning in their Dow Corning SH200 series.

### AMINOSILICONE

The compositions of the present invention comprise an amino silicone having less than about 0.5% nitrogen by weight of the aminosilicone, preferably less than about 0.2%, more preferably less than about 0.1%, in view of friction reduction benefit. It has been surprisingly found that higher levels of nitrogen (amine functional groups) in the amino silicone tend to result in less friction reduction, and consequently less conditioning benefit from the aminosilicone. The aminosilicone useful herein preferably has at least one silicone block with greater than 200 siloxane units, in view of friction reduction benefit. The aminosilicones useful herein include, for example, quaternized aminosilicone and non-quatemized aminosilicone.

Preferably, the aminosilicone useful herein are those which conform to the general formula (I):

(R₁)ₐG₃₋ₐ-Si-(-OSiG₂)ₙ-(-OSiG_{b}(R₁)_{2-b})ₘ-O-SiG₃₋ₐ(R₁)ₐ

wherein G is hydrogen, phenyl, hydroxy, or C₁-C₈ alkyl, preferably methyl; a is an integer having a value from 1 to 3, preferably 1; b is 0, 1 or 2, preferably 1; n is a number from 1 to 2,000 , preferably from 100 to 1,800, more preferably from 300 to 800, still more preferably 500-600; m is an integer from 0 to 1,999, preferably from 0-10, more preferably m is 0; R₁ is a monovalent radical conforming to the general formula CqH_{2q}L, wherein q is an integer having a value from 2 to 8 and L is selected from the following groups: -N(R₂)CH₂-CH₂-N(R₂)₂; -N(R₂)₂; -N(R₂)₃A⁻; -N(R₂)CH₂-CH₂-NR₂H₂A⁻; wherein R₂ is hydrogen, phenyl, benzyl, or a saturated hydrocarbon radical, preferably an alkyl radical from about C₁ to about C₂₀; A⁻ is a halide ion. L is preferably -N(CH₃)₂ or -NH₂, more preferably - NH₂.

The amino silicone of the above formula is used at levels by weight of the composition of from about 0.1% to about 5%, more preferably from about 0.2% to about 2%, still more preferably from about 0.20% to about 1.0%, even more preferably about 0.30% to about 0.8%

One highly preferred amino silicones are those corresponding to formula (I) wherein m=0, a=1, q=3, G=methyl, n is preferably from about 1400 to about 1700, more preferably around 1600; and L is -N(CH₃)₂ or NH₂, more preferably -NH₂. Another further highly preferred amino silicones are those corresponding to formula (I) wherein m=0, a=1, q=3, G=methyl, n is preferably from about 400 to about 800, more preferably about 500 to around 600; and L is -N(CH₃)₂ or -NH₂, more preferably -NH₂. Such highly preferred amino silicones can be called as terminal aminosilicones, as one or both ends of the silicone chain are terminated by nitrogen containing group. It is also surprisingly found that, such terminal aminosilicones provide improved friction reduction compared to graft aminosilicones.

Another example of aminosilicone useful herein includes, for example, quaternized aminosilicone having a tradename KF8020 available from Shinetsu.

The above aminosilicones, when incorporated into the composition, can be mixed with solvent having a lower viscosity. Such solvents include, for example, polar or non-polar, volatile or non-volatile oils. Such oils include, for example, silicone oils, hydrocarbons, and esters. Among such a variety of solvents, preferred are those selected from the group consisting of non-polar, volatile hydrocarbons, volatile cyclic silicones, non-volatile linear silicones, and mixtures thereof. The non-volatile linear silicones useful herein are those having a viscosity of from about 1 to about 20,000 mm²/s, preferably from about 20 to about 10,000 mm²/s at 25°C. Among the preferred solvents, highly preferred are non-polar, volatile hydrocarbons, especially non-polar, volatile isoparaffins, in view of reducing the viscosity of the aminosilicones and providing improved hair conditioning benefits such as reduced friction on dry hair. Such mixtures have a viscosity of preferably from about 1,000mPa·s to about 100,000mPa·s, more preferably from about 5,000mPa·s to about 50,000mPa·s.

### SILICONE COPOLYMER EMULSION

Compositions of the present invention also comprise a silicone copolymer emulsion with an internal phase viscosity of greater than about 100 x 10⁶ mm²/s. The silicone copolymer emulsion is present in an amount of from about 0.1% to about 15%, preferably from about 0.3% to about 10%, and more preferably from about 0.5% to about 5%, by weight of the composition, in view of providing clean feel. It is believed that this silicone copolymer emulsion, together with the above polyalkylsiloxane mixture and aminosilicone, can form a film on hair which provides enhanced clean feel. It is also believed that this silicone copolymer emulsion additionally provides body/fullness to hair, together with the above polyalkylsiloxane mixture and aminosilicone, by forming a film on hair.

The silicone copolymer emulsion has a viscosity at 25°C of greater than about 100 x 10⁶ mm²/s, preferably greater than about 120 x 10⁶ mm²/s, more preferably greater than about 150 x 10⁶ mm²/s. The silicone copolymer emulsion has a viscosity at 25°C of, preferably less than about 1000 x 10⁶ mm²/s, more preferably less than about 500 x 10⁶ mm²/s, and even more preferably less than about 300 x 10⁶ mm²/s. To measure the internal phase viscosity of the silicone copolymer emulsion, one must first break the polymer from the emulsion. By way of example, the following procedure can be used to break-the polymer from the emulsion: 1) add 10 grams of an emulsion sample to 15 milliliters of isopropyl alcohol; 2) mix well with a spatula; 3) decant the isopropyl alcohol; 4) add 10 milliliters of acetone and knead polymer with spatula; 5) decant the acetone; 6) place polymer in an aluminum container and flatten/dry with a paper towel; and 7) dry for two hours in an 80 °C. The polymer can then be tested using any known rheometer, such as, for example, a CarriMed, Haake, or Monsanto rheometer, which operates in the dynamic shear mode. The internal phase viscosity values can be obtained by recording the dynamic viscosity (n') at a 9.900*10⁻³ Hz frequency point. The average particle size of the emulsions is preferably less than about 1 micron, more preferably less than about 0.7 micron.

The silicone copolymer emulsions of the present invention comprise a silicone copolymer, at least one surfactant, and water.

The silicone copolymer results from the addition reaction of the following two materials in the presence of a metal containing catalyst:
(a) a polysiloxane with reactive groups on both termini, represented by formula (III) 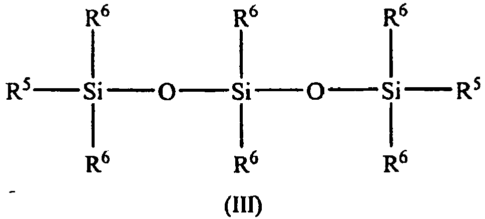 wherein:
   R⁵ is a group capable of reacting by chain addition reaction such as, for example, a hydrogen atom, an aliphatic group with ethylenic unsaturation (i.e., vinyl, allyl, or hexenyl), a hydroxyl group, an alkoxyl group (i.e., methoxy, ethoxy, or propoxy), an acetoxyl group, or an amino or alkylamino group; preferably, R⁵ is hydrogen or an aliphatic group with ethylenic unsaturation; more preferably, R⁵ is hydrogen;
   R⁶ is alkyl, cycloalkyl, aryl, or alkylaryl and may include additional functional groups such as ethers, hydroxyls, amines, carboxyls, thiols esters, and sulfonates; preferably, R⁶ is methyl. Optionally, a small mole percentage of the groups may be reactive groups as described above for R⁵, to produce a polymer which is substantially linear but with a small amount of branching. In this case, preferably the level of R⁶ groups equivalent to R⁵ groups is less than about 10% on a mole percentage basis, and more preferably less than about 2%;
   n is a whole number such that the polysiloxane of formula (III) has a viscosity of from about 1 mm²/s to about 1 x 10⁶ mm²/s;
   and,
(b) at least one silicone compound or non-silicone compound comprising at least one or at most two groups capable of reacting with the R₅ groups of the polysiloxane in formula (III); preferably, the reactive group is an aliphatic group with ethylenic unsaturation.

The metal containing catalysts used in the above described reactions are often specific to the particular reaction. Such catalysts are known in the art. Generally, they are materials containing metals such as platinum, rhodium, tin, titanium, copper, lead, etc.

The mixture used to form the emulsion also contains at least one surfactant. This can include non-ionic surfactants, cationic surfactants, anionic surfactants, alkylpolysaccharides, amphoteric surfactants, and the like. The above surfactants can be used individually or in combination.

An exemplary method of making the silicone copolymer emulsions described herein comprises the steps of 1) mixing materials (a) described above with material (b) described above, followed by mixing in an appropriate metal containing catalyst, such that material (b) is capable of reacting with material (a) in the presence of the metal containing catalyst; 2) further mixing in at least one surfactant and water; and 3) emulsifying the mixture. Methods of making such silicone copolymer emulsions are disclosed in U.S. Patent No. 6,013,682; PCT Application No. WO01/58986 A1; and European Patent Application No. EP0874017 A2.

Commercially available example of highly preferred silicone copolymer emulsion is an emulsion of about 60-70% of divinyldimethicone / dimethicone copolymer having an internal phase viscosity of minimum 120 x 10⁶ mm²/s, available from Dow Coming with a tradename HMW2220.

### CATIONIC SURFACTANT SYSTEM

The composition of the present invention comprises a cationic surfactant system. The cationic surfactant system can be one cationic surfactant or a mixture of two or more cationic surfactants. The cationic surfactant system is included in the composition at a level by weight of from about 0.1 % to about 10%, preferably from about 0.5% to about 8%, more preferably from about 0.8% to about 5%, still more preferably from about 1.0% to about 4%, in view of desired gel network formation and wet conditioning benefits.

A variety of cationic surfactants including mono- and di-alkyl chain cationic surfactants can be used in the compositions of the present invention. The cationic surfactant system of the present invention preferably contains mono-alkyl chain cationic surfactants in view of providing desired gel matrix and wet conditioning benefits. The mono-alkyl cationic surfactants are those having one long alkyl chain which has from 12 to 22 carbon atoms, preferably from 16 to 22 carbon atoms, more preferably C18-22 alkyl group, in view of providing balanced wet conditioning benefits. The remaining groups attached to nitrogen are independently selected from an alkyl group of from 1 to about 4 carbon atoms or an alkoxy, polyoxyalkylene, alkylamido, hydroxyalkyl, aryl or alkylaryl group having up to about 4 carbon atoms. Such mono-alkyl cationic surfactants include, for example, mono-alkyl quaternary ammonium salts and mono-alkyl amines. Mono-alkyl quaternary ammonium salts include, for example, those having a non-functionalized long alkyl chain. Mono-alkyl amines include, for example, mono-alkyl amidoamines and salts thereof.

Mono-long alkyl quaternized ammonium salts useful herein are those having the formula (II): 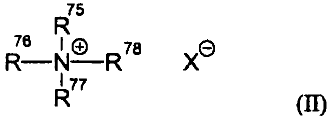 wherein one of R⁷⁵, R⁷⁶, R⁷⁷ and R⁷⁸ is selected from an alkyl group of from 12 to 30 carbon atoms or an aromatic, alkoxy, polyoxyalkylene, alkylamido, hydroxyalkyl, aryl or alkylaryl group having up to about 30 carbon atoms; the remainder of R⁷⁵, R⁷⁶, R⁷⁷ and R⁷⁸ are independently selected from an alkyl group of from 1 to about 4 carbon atoms or an alkoxy, polyoxyalkylene, alkylamido, hydroxyalkyl, aryl or alkylaryl group having up to about 4 carbon atoms; and X is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The alkyl groups can contain, in addition to carbon and hydrogen atoms, ether and/or ester linkages, and other groups such as amino groups. The longer chain alkyl groups, e.g., those of about 12 carbons, or higher, can be saturated or unsaturated. Preferably, one of R⁷⁵, R⁷⁶, R⁷⁷ and R⁷⁸ is selected from an alkyl group of from 12 to 30 carbon atoms, more preferably from 16 to 22 carbon atoms, still more preferably from 18 to 22 carbon atoms, even more preferably 22 carbon atoms; the remainder of R⁷⁵, R⁷⁶, R⁷⁷ and R⁷⁸ are independently selected from CH₃, C₂H₅, C₂H₄OH, and mixtures thereof; and X is selected from the group consisting of Cl, Br, CH₃OSO₃, C₂H₅OSO₃, and mixtures thereof.

Examples of preferred mono-long alkyl quaternized ammonium salt cationic surfactants include: behenyl trimethyl ammonium salt; stearyl trimethyl ammonium salt; cetyl trimethyl ammonium salt; and hydrogenated tallow alkyl trimethyl ammonium salt. Among them, highly preferred are behenyl trimethyl ammonium salt and stearyl trimethyl ammonium salt.

Mono-alkyl amines are also suitable as cationic surfactants. Primary, secondary, and tertiary fatty amines are useful. Particularly useful are tertiary amido amines having an alkyl group of from about 12 to about 22 carbons. Exemplary tertiary amido amines include: stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, diethylaminoethylstearamide. Useful amines in the present invention are disclosed in U.S. Patent 4,275,055, Nachtigal, et al. These amines can also be used in combination with acids such as ℓ-glutamic acid, lactic acid, hydrochloric acid, malic acid, succinic acid, acetic acid, fumaric acid, tartaric acid, citric acid, ℓ-glutamic hydrochloride, maleic acid, and mixtures thereof; more preferably ℓ-glutamic acid, lactic acid, citric acid. The amines herein are preferably partially neutrilized with any of the acids at a molar ratio of the amine to the acid of from about 1 : 0.3 to about 1 : 2, more preferably from about 1 : 0.4 to about 1 : 1.

Although the mono-alkyl chain cationic surfactants are preferred, other cationic surfactants such as di-alkyl chain cationic surfactants may also be used alone, or in combination with the mono-alkyl chain cationic surfactants. Such di-alkyl chain cationic surfactants include, for example, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride. Such di-alkyl chain cationic surfactants also include, for example, tallowalkyl (2-ethylhexyl) dimethyl ammonium methosulfate which is available, for example, from Akzo Nobel with a tradename Arquad HTL8-MS.

### HIGH MELTING POINT FATTY COMPOUND

The high melting point fatty compound useful herein have a melting point of 25°C or higher, and is selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than 25°C. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

Among a variety of high melting point fatty compounds, fatty alcohols are preferably used in the composition of the present invention. The fatty alcohols useful herein are those having from about 14 to about 30 carbon atoms, preferably from about 16 to about 22 carbon atoms. These fatty alcohols are saturated and can be straight or branched chain alcohols. Preferred fatty alcohols include, for example, cetyl alcohol, stearyl alcohol, behenyl alcohol, and mixtures thereof. -

High melting point fatty compounds of a single compound of high purity are preferred. Single compounds of pure fatty alcohols selected from the group of pure cetyl alcohol, stearyl alcohol, and behenyl alcohol are highly preferred. By "pure" herein, what is meant is that the compound has a purity of at least about 90%, preferably at least about 95%. These single compounds of high purity provide good rinsability from the hair when the consumer rinses off the composition.

The high melting point fatty compound is included in the composition at a level of from about 1% to about 20%, preferably from about 3% to about 10%, more preferably from about 4% to about 8% by weight of the composition, in view of providing improved conditioning benefits such as slippery feel during the application to wet hair, softness and moisturized feel on dry hair.

### GEL MATRIX

The composition of the present invention comprises a gel matrix. The gel matrix comprises a cationic surfactant, a high melting point fatty compound, and an aqueous carrier.

The gel matrix is suitable for providing various conditioning benefits such as slippery feel during the application to wet hair and softness and-moisturized feel on dry hair. In view of providing the above gel matrix, the cationic surfactant and the high melting point fatty compound are contained at a level such that the weight ratio of the cationic surfactant to the high melting point fatty compound is in the range of, preferably from about 1:1 to about 1:10, more preferably from about 1:1 to about 1:6.

For forming gel matrix, it is preferred to prepare the composition by the following method:

Water is typically heated to at least about 70°C, preferably between about 80°C and about 90°C. The cationic surfactant and the high melting point fatty compound are combined with the water to form a mixture. The temperature of the mixture is preferably maintained at a temperature higher than both the melting temperature of the cationic surfactant and the melting temperature of the high melting point fatty compound, and the entire mixture is homogenized. After mixing until no solids are observed, the mixture is gradually cooled (e.g., at a rate of from about 1°C/minute to about 5°C/minute) to a temperature below 60°C, preferably less than about 55°C. During this gradual cooling process, a significant viscosity increase is observed at between about 55°C and about 75°C. This indicates the formation of gel matrix. The high molecular weight water-soluble cationic polymer can be added to the mixture with agitation at about 55°C, or prior to the cooling down. Additional components are then combined with the gel matrix, and cooled to room temperature.

Preferably, the present invention comprises, by weight of the hair care composition, from about 60% to about 99%, preferably from about 70% to about 95%, and more preferably from about 80% to about 95% of a gel matrix, to which optional ingredients such as silicones can be added. The composition containing the above amount of gel matrix is typically characterized by rheology at 950s-1 of from about 40 Pa to about 600 Pa, preferably from about 50 Pa to about 500 Pa, and more preferably from about 70 Pa to about 400Pa, as measured at 26.7 °C, by means of TA AR1000 rheometer at shear rate from 0.1s-1 to 1100s-1 with the duration of 1 minutes. Although the composition of the present invention can contain a thickening polymer, the composition of the present invention can have the above rheology with the presence of any thickening polymer.

### AQUEOUS CARRIER

The conditioning composition of the present invention comprises an aqueous carrier. The level and species of the carrier are selected according to the compatibility with other components, and other desired characteristic of the product.

The carrier useful in the present invention includes water and water solutions of lower alkyl alcohols and polyhydric alcohols. The lower alkyl alcohols useful herein are monohydric alcohols having 1 to 6 carbons, more preferably ethanol and isopropanol. The polyhydric alcohols useful herein include propylene glycol, hexylene glycol, glycerin, and propane diol.

Preferably, the aqueous carrier is substantially water. Deionized water is preferably used. Water from natural sources including mineral cations can also be used, depending on the desired characteristic of the product. Generally, the compositions of the present invention comprise from about 20% to about 99%, preferably from about 30% to about 95%, and more preferably from about 80% to about 95% water.

### ADDITIONAL COMPONENTS

The composition of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from about 0.001% to about 10%, preferably up to about 5% by weight of the composition.

A wide variety of other additional components can be formulated into the present compositions. These include: low melting point oils having a melting point of less than 25°C including, for example, unsaturated fatty alcohols such as oleyl alcohol and ester oils such as pentaerythritol ester oils; cationic conditioning polymers including, for example, cationic celluloses and cationic guar gums; polyethylene glycols; other conditioning agents such as hydrolysed collagen with tradename Peptein 2000 available from Hormel, vitamin E with tradename Emix-d available from Eisai, panthenol available from Roche, panthenyl ethyl ether available from Roche, hydrolysed keratin, proteins, plant extracts, and nutrients; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents, such as any of the FD&C or D&C dyes; perfumes; and sequestering agents, such as disodium ethylenediamine tetra-acetate; ultraviolet and infrared screening and absorbing agents such as octyl salicylate; and antidandruff agents such as zinc pyrithione and salicylic acid.

### PRODUCT FORMS

The hair care compositions can be formulated into a variety of product forms, including shampoos, conditioners (both rinse-off and leave-on versions), styling products, and the like. In one embodiment, the hair care compositions include only hair conditioners and do not include shampoos or styling products.

The conditioning compositions of the present invention can be in the form of rinse-off products or leave-on products, and can be formulated in a wide variety of product forms, including but not limited to creams, gels, emulsions, mousses and sprays.

The conditioning composition of the present invention is especially suitable for rinse-off hair conditioner. Such compositions are preferably used by following steps:
(i) after shampooing hair, applying to the hair an effective amount of the conditioning compositions for conditioning the hair; and
(ii) then rinsing the hair.

### EXAMPLES

The following examples 1-5, 7 and 8 further describe and demonstrate embodiments within the scope of the present invention. The examples are given solely for the purpose of illustration and are not to be construed as limitations of the present invention, as many variations thereof are possible without departing from the spirit and scope of the invention. Where applicable, ingredients are identified by chemical or CTFA name, or otherwise defined below.

### [Compositions]

| Components | Ex.1 | Ex.2 | Ex.3 | Ex.4 | Ex.5 | Ex.6 | Ex.7 | Ex.8 | Ex.9 |
|---|---|---|---|---|---|---|---|---|---|
| Polyalkylsiloxane-1 *1 | 4.0 | 4.0 | 4.0 | 4.0 | 4.0 | - | 0.8 | 4.0 | - |
| Polyalkylsiloxane-2 *2 | - | - | - | - | - | 3.0 | - | - | 8.0 |
| Aminosilicone-1 *3 | 0.5 | 0.5 | 0.5 | - | 0.25 | 0.5 | 0.2 | - | 1.0 |
| Aminosilicone-2 *4 | - | - | - | 1.0 | - | - | - | - | - |
| Isoparaffin | - | - | - | 0.43 | - | - | - | - | - |
| Aminosilicone-3 *S | - | - | - | - | - | - | - | 0.5 | - |
| Silicone copolymer emulsion *6 | 2.0 | 2.0 | 2.0 | 2.0 | 2.0 | 2.0 | 3.0 | 2.0 | 4.0 |
| Tallow dimethyl ammonium chloride *7 | - | - | 0.64 | - | - | - | - | - | - |
| Behenyl trimethyl ammonium chloride | 3.38 | - | 0.96 | - | 2.25 | 3.38 | 3.38 | - | - |
| Behenyl trimethyl ammonium methosulfate | - | 1.8 | - | 1.8 | - | - | - | 1.8 | 1.8 |
| Isopropyl alcohol | 0.899 | 0.45 | 0.236 | 0.45 | 0.598 | 0.899 | 0.899 | 0.45 | 0.45 |
| Cetyl alcohol | 2.32 | 1.49 | 1.30 | 1.49 | 1.9 | 2.32 | 2.32 | 1.49 | 1.49 |
| Stearyl alcohol | 4.18 | 3.71 | 3.25 | 3.71 | 4.6 | 4.18 | 4.18 | 3.71 | 3.71 |
| Preservatives | 0.53 | 0.53 | 0.53 | 0.53 | 0.53 | 0.53 | 0.53 | 0.53 | 0.53 |
| Perfume | 0.4 | 0.4 | 0.4 | 0.4 | 0.35 | 0.25 | 0.25 | 0.25 | 0.4 |
| Panthenol | 0.05 | 0.05 | 0.05 | 0.05 | - | - | 0.05 | 0.05 | 0.05 |
| Panthenyl ethyl ether | 0.03 | 0.03 | 0.03 | 0.03 | - | - | 0.05 | 0.05 | 0.03 |
| Sodium hydroxide | 0.014 | - | - | - | - | 0.014 | 0.014 | - | - |
| Deionized Water | q.s. to 100% | | | | | | | | |

| | | | | | | | | | |
|---|---|---|---|---|---|---|---|---|---|
| Definitions of Components *1 Polyalkylsiloxane-1: a blend of 15% of dimethicone having a viscosity of 18,000,000mPa•s and 85% of dimethicone having a viscosity of 200mPa•s available from GE Toshiba, the blend having a viscosity of about 20,000mPa•s *2 Polyalkylsiloxane-2: a blend of 15% of dimethicone having a viscosity of 500,000mPa•s and 85% of dimethicone having a viscosity of 200mPa•s available from GE Toshiba, the blend having a viscosity of about 1,500mPa•s (not according ingredient (a) of the invention) *3 Aminosilicone-1: Terminal aminosilicone which is available from GE having a viscosity of about 10,000mPa•s, and having following formula: (R₁)ₐG₃₋ₐ-Si-(-OSiG₂)ₙ-(-OSiG_{b}(R₁)_{2-b})ₘ-O-SiG₃₋ₐ(R₁)ₐ wherein G is methyl; a is an integer of 1; n is a number from 400 to about 600; m is an integer of 0; R₁ is a monovalent radical conforming to the general formula CqH_{2q}L, wherein q is an integer of 3 and L is -NH₂ *4 Aminosilicone-2: Terminal aminosilicone which is available from GE having a viscosity of about 230,000mPa•s, and having following formula: (R₁)ₐG₃₋ₐ-Si-(-OSiG₂)ₙ-(-OSiG_{b}(R₁)_{2-b})ₘ-O-SiG₃₋ₐ(R₁)ₐ wherein G is methyl; a is an integer of 1; n is a number from 1,500 to about 1,700; m is an integer of 0; R₁ is a monovalent radical conforming to the general formula CqH_{2q}L, wherein q is an integer of 3 and L is -NH₂ *5 Aminosilicone-3: KF8020 available from Shinetsu *6 Silicone copolymer emulsion: an emulsion of about 60-70% of divinyldimethicone / dimethicone copolymer having an internal phase viscosity of minimum 120 x 10⁶ mm²/s, available from Dow Coming with a tradename HMW2220 *7 Tallow dimethyl ammonium chloride: 84% Aqueous mixture of Hydrogenated tallowalkyl (2-ethylhexyl) dimethyl quaternary ammonium methosulfate available with a tradename Arquad HTL8-MS from Akzo Nobel | | | | | | | | | |

### Method of Preparation

The conditioning compositions of "Ex. 1" through "Ex. 9" as shown above can be prepared by any conventional method well known in the art. They are suitably made as follows:

Cationic surfactants and high melting point fatty compounds are added to water with agitation, and heated to about 80°C. The mixture is cooled down to about 55°C. Silicone compounds are added to the mixture with agitation at about 55°C. If included, other components such as perfumes and preservatives are added to the mixture with agitation. Then the mixture is cooled down to room temperature.

Examples 1 through 9 are hair conditioning compositions of the present invention which are particularly useful for rinse-off use. The embodiments disclosed and represented by the previous "Ex. 1" through "Ex. 9" have many advantages. For example, they provide clean feel and/or reduced tackiness, while providing such improved conditioning benefits such as smooth feel and reduced friction.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as-"40 mm" is intended to mean "about 40 mm."

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

## Claims

1. A hair conditioning composition comprising by weight:
(a) from 0.5% to 10% of a polyalkylsiloxane mixture comprising:
(i) a first polyalkylsiloxane which is non-volatile, contains 0 % of amino groups, and has a viscosity of from 10,000,000 mm²· s⁻¹to 20,000,000 mm²· s⁻¹; and
(ii)a second polyalkylsiloxane which is non-volatile, contains 0 % of amino groups, and has a viscosity of from 20 mm²· s⁻¹ to 350 mm²· s⁻¹;
(b) from 0.1% to 5% of an aminosilicone having less than 0.5% nitrogen by weight of the aminosilicone;
(c) from 0.1% to 5% of a silicone copolymer emulsion with an internal phase viscosity of greater than 100 x 10⁶ mm²/s, as measured at 25°C; and
(d) an aqueous carrier;
wherein the composition contains 1% or less of anionic surfactants and anionic polymers; and wherein the weight ratio of the first polysiloxane to the second polysiloxane is from 1:2 to 1:9.

2. The hair conditioning composition of Claim 1 wherein the polyalkylsiloxane mixture has a viscosity of from 500 mm²· s⁻¹ to 100,000 mm²· s⁻¹.

3. The hair conditioning composition of Claim 1 wherein the polyalkylsiloxane mixture and the aminosilicone are contained at a level such that the weight ratio of the polyalkylsiloxane mixture to the aminosilicone is from 1:1 to 20:1.

4. The hair conditioning composition of Claim 1 wherein the polyalkylsiloxane mixture, aminosilicone and silicone copolymer emulsion are contained at a level such that the weight ratio of (i) a total of amount of the polyalkylsiloxane mixture and aminosilicone to (ii) the silicone copolymer emulsion is from 1:4 to 10:1.

5. The hair conditioning composition of Claim 1 wherein the aminosilicone has at least one silicone block with greater than 200 siloxane units.

6. The hair conditioning composition of Claim 1 wherein the aminosilicone has a formula:
(R₁)ₐG₃₋ₐ-Si-(-OSiG₂)ₙ-(-OSiG_{b}(R₁)_{2-b})ₘ-O-SiG₃₋ₐ(R₁)a
wherein G is hydrogen, phenyl, hydroxy, or C₁-C₈ alkyl; a is an integer having a value from 1 to 3; b is 0, 1 or 2; n is a number from 1 to 2,000; m is an integer from 0 to 1,999; R₁ is a monovalent radical conforming to the general formula CqH_{2q}L, wherein q is an integer having a value from 2 to 8 and L is selected from the following groups: -N(R₂)CH₂-CH₂-N(R₂)₂; -N(R₂)₂; -N(R₂)₃A⁻; -N(R₂)CH₂-CH₂-NR₂H₂A⁻; wherein R₂ is hydrogen, phenyl, benzyl, or a saturated hydrocarbon radical; A is a halide ion.

7. The hair conditioning composition of Claim 7 wherein the aminosilicone has a formula:
(R₁)ₐG₃₋ₐ-Si-(-OSiG₂)ₙ-(-OSiG_{b}(R₁)₂-_{b})ₘ-O-SiG₃₋ₐ(R₁)ₐ
wherein G is hydrogen, phenyl, hydroxy, or C₁-C₈ alkyl; a is 1; n is a number from 100 to 1,800; m is 0; R₁ is a monovalent radical conforming to the general formula CqH_{2q}L, wherein q is an integer having a value from 2 to 8 and L is -N(R₂)₂; wherein R₂ is hydrogen, phenyl, benzyl, or a saturated hydrocarbon radical; and A is a halide ion.

8. The hair conditioning composition of Claim 8 wherein L is -N(CH₃)₂ or -NH₂.

9. The composition of Claim 1, wherein the silicone copolymer emulsion has an internal phase viscosity of greater than 120 x 10⁶ mm²/s, as measured at 25°C.

10. The composition of Claim 1, wherein the silicone copolymer emulsion comprises a silicone copolymer, at least one surfactant, and water, wherein the silicone copolymer comprises a polysiloxane having reactive groups on both termini, and corresponding to the following formula: 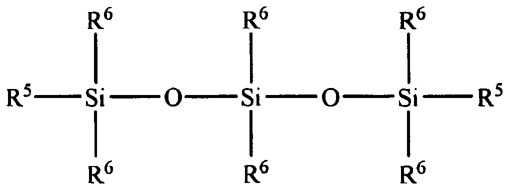 wherein R⁵ is selected from the group consisting of a hydrogen atom, an aliphatic group with ethylenic unsaturation, a hydroxyl group, an alkoxyl group, an acetoxyl group, an amino or alkylamino group, and mixtures thereof; and R⁶ is selected from the group consisting of an alkyl, a cycloalkyl, an aryl, an alkylaryl, ethers, hydroxyls, amines, carboxyls, thiols esters, sulfonates, and mixtures thereof.

11. The hair conditioning composition of Claim 1 further comprising: from 0.1% to 10% by weight of the composition of a cationic surfactant; and from 0.1% to 20% by weight of the composition of a high melting point fatty compound.

12. The hair care composition of Claim 1, wherein the composition is a rinse-off conditioner.

## Patentansprüche

1. Haarkonditionierungszusammensetzung, umfassend in Gewichtsprozent:
(a) von 0,5 % bis 10 % eine Polyalkylsiloxanmischung, umfassend:
(i) ein erstes Polyalkylsiloxan, das nichtflüchtig ist, 0 % Aminogruppen enthält und eine Viskosität von 10.000.000 mm²·s⁻¹ bis 20.000.000 mm²·s⁻¹ aufweist; und
(ii) ein zweites Polyalkylsiloxan, das nichtflüchtig ist, 0 % Aminogruppen enthält und eine Viskosität von 20 mm²·s⁻¹ bis 350 mm²·s⁻¹ aufweist;
(b) von 0,1 % bis 5 % ein Aminosilikon, das zu weniger als 0,5 Gew.-% des Aminosilikons Stickstoff aufweist;
(c) von 0,1 % bis 5 % eine Silikoncopolymeremulsion mit einer internen Phasenviskosität von mehr als 100 x 10⁶ mm²/s, wie bei 25 °C gemessen; und
(d) einen wässrigen Träger.
wobei die Zusammensetzung zu 1 % oder weniger anionische Tenside und anionische Polymere enthält;
und wobei das Gewichtsverhältnis des ersten Polysiloxans zum zweiten Polysiloxan von 1:2 bis 1:9 beträgt.

2. Haarkonditionierungszusammensetzung nach Anspruch 1, wobei die Polyalkylsiloxanmischung eine Viskosität von 500 mm²·s⁻¹ bis 100.000 mm²·s⁻¹ aufweist.

3. Haarkonditionierungszusammensetzung nach Anspruch 1, wobei die Polyalkylsiloxanmischung und das Aminosilikon in einer solchen Konzentration enthalten sind, dass das Gewichtsverhältnis der Polyalkylsiloxanmischung zu dem Aminosilikon von 1:1 1 bis 20:1 beträgt.

4. Haarkonditionierungszusammensetzung nach Anspruch 1, wobei die Polyalkylsiloxanmischung, das Aminosilikon und die Silikoncopolymeremulsion in einer solchen Konzentration enthalten sind, dass das Gewichtsverhältnis von (i) einer Gesamtmenge der Polyalkylsiloxanmischung und des Aminosilikons zu (ii) der Silikoncopolymeremulsion von 1:4 bis 10:1 1 beträgt.

5. Haarkonditionierungszusammensetzung nach Anspruch 1, wobei das Aminosilikon mindestens einen Silikonblock mit mehr als 200 Siloxaneinheiten aufweist.

6. Haarkonditionierungszusammensetzung nach Anspruch 1, wobei das Aminosilikon die folgende Formel aufweist:
(R₁)aG₃₋ₐ-Si-(-OSiG₂)ₙ-(-OSiG_{b}(R₁)_{2-b})ₘ-O-SiG₃₋ₐ(R₁)ₐ
wobei G Wasserstoff, Phenyl, Hydroxy oder C₁-C₈-Alkyl ist; a eine ganze Zahl mit einem Wert von 1 bis 3 ist; b 0, 1 oder 2 ist; n eine Zahl von 1 bis 2.000 ist; m eine ganze Zahl von 0 bis 1.999 ist; R₁ ein einwertiges Radikal entsprechend der allgemeinen Formel CqH_{2q}L ist, wobei q eine ganze Zahl mit einem Wert von 2 bis 8 ist und L aus den folgenden Gruppen ausgewählt ist: -N(R₂)CH₂-CH₂-N(R₂)₂; -N(R₂)₂; -N(R₂)₃A ; -N(R₂)CH₂-CH₂-NR₂H₂A⁻; wobei R₂ Wasserstoff, Phenyl, Benzyl oder ein gesättigtes Kohlenwasserstoffradikal ist; A ein Halogenidion ist.

7. Haarkonditionierungszusammensetzung nach Anspruch 7, wobei das Aminosilikon die folgende Formel aufweist:
(R₁)ₐG₃₋ₐ-Si-(-OSiG₂)ₙ-(-OSiG_{b}(R₁)_{2-b})ₘ-O-SiG₃₋ₐ(R₁)ₐ
wobei G Wasserstoff, Phenyl, Hydroxy oder C₁-C₈-Alkyl ist; a 1 ist; n eine Zahl von 100 bis 1.800 ist; m 0 ist; R₁ ein einwertiges Radikal entsprechend der allgemeinen Formel CqH₂qL ist, wobei q eine ganze Zahl mit einem Wert von 2 bis 8 ist und L -N(R₂)₂ ist; wobei R₂ Wasserstoff, Phenyl, Benzyl oder ein gesättigtes Kohlenwasserstoffradikal ist; und A ein Halogenidion ist.

8. Haarkonditionierungszusammensetzung nach Anspruch 8, wobei L -N(CH₃)₂ oder -NH₂ ist.

9. Zusammensetzung nach Anspruch 1, wobei die Silikoncopolymeremulsion eine interne Phasenviskosität von mehr als 120 x 10⁶ mm²/s aufweist, wie bei 25 °C gemessen.

10. Zusammensetzung nach Anspruch 1, wobei die Silikoncopolymeremulsion ein Silikoncopolymer, mindestens ein Tensid und Wasser, umfasst, wobei das Silikoncopolymer ein Polysiloxan mit reaktionsfähigen Gruppen an beiden Enden umfasst und der folgenden Formel entspricht: 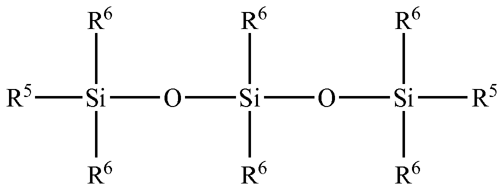 wobei R⁵ ausgewählt ist aus der Gruppe bestehend aus einem Wasserstoffatom, einer aliphatischen Gruppe mit ethylenischer Nichtsättigung, einer Hydroxylgruppe, einer Alkoxylgruppe, einer Acetoxylgruppe, einer Amino- oder Alkylaminogruppe und Mischungen davon; und R⁶ ausgewählt ist aus der Gruppe bestehend aus einem Alkyl, einem Cycloalkyl, einem Aryl, einem Alkylaryl, Ethern, Hydroxylen, Aminen, Carboxylen, Thiolestern, Sulfonaten und Mischungen davon.

11. Haarkonditionierungszusammensetzung nach Anspruch 1, ferner umfassend: von 0,1 Gew.-% bis 10 Gew.-% der Zusammensetzung ein kationisches Tensid; und von 0,1 Gew.-% bis 20 Gew.-% der Zusammensetzung eine Fettverbindung mit hohem Schmelzpunkt.

12. Haarpflegezusammensetzung nach Anspruch 1, wobei die Zusammensetzung ein Konditioniermittel zum Ausspülen ist.

## Revendications

1. Composition de conditionnement des cheveux comprenant en poids :
(a) de 0,5 % à 10 % d'un mélange de polyalkylsiloxanes comprenant :
(i) un premier polyalkylsiloxane qui est non volatil, contient 0 % de groupes amino, et a une viscosité allant de 100 000 mm²· s⁻¹ à 20 000 000 mm²· s⁻¹ ; et
(ii) un deuxième polyalkylsiloxane qui est non volatil, contient 0 % de groupes amino, et a une viscosité allant de 20 mm²· s⁻¹ à 350 mm²· s⁻¹ ;
(b) de 0,1 % à 5 % d'une aminosilicone ayant moins de 0,5 % d'azote en poids de l'aminosilicone ;
(c) de 0,1 % à 5 % d'une émulsion de copolymère de silicone avec une viscosité de phase interne supérieure à 100 x 10⁶ mm²/s, telle que mesurée à 25 °C ; et
(d) un véhicule aqueux.
où la composition contient 1 % ou moins d'agents tensioactifs anioniques et de polymères anioniques ;
et dans laquelle le rapport pondéral du premier polysiloxane sur le deuxième polysiloxane va de 1:2 à 1:9.

2. Composition de conditionnement des cheveux selon la revendication 1, dans laquelle le mélange de polyalkylsiloxanes a une viscosité allant de 500 mm²· s⁻¹ à 100 000 mm²· s⁻¹.

3. Composition de conditionnement des cheveux selon la revendication 1, dans laquelle le mélange de polyalkylsiloxanes et l'aminosilicone sont contenus à un taux tel que le rapport pondéral du mélange de polyalkylsiloxanes sur l'aminosilicone va de 1:1 à 20:1.

4. Composition de conditionnement des cheveux selon la revendication 1, dans laquelle le mélange de polyalkylsiloxanes, l'aminosilicone et l'émulsion de copolymère de silicone sont contenus à un taux tel que le rapport pondéral de (i) la quantité totale du mélange de polyalkylsiloxanes et de l'aminosilicone sur (ii) l'émulsion de copolymère de silicone va de 1:4 à 10:1.

5. Composition de conditionnement des cheveux selon la revendication 1, dans laquelle l'aminosilicone a au moins un bloc de silicone avec plus de 200 motifs siloxane.

6. Composition de conditionnement des cheveux selon la revendication 1, dans laquelle l'aminosilicone est de formule :
(R₁)ₐG₃₋ₐ-Si-(-OSiG₂)ₙ-(-OSiG_{b}(R₁)_{2-b})ₘ-O-SiG₃₋ₐ(R₁)ₐ
dans laquelle G est un hydrogène, un phényle, un hydroxy, ou un alkyle en C₁ à C₈ ; A est nombre entier ayant une valeur allant de 1 à 3 ; b est 0, 1 ou 2 ; n est un nombre de 1 à 2000 ; m est un nombre entier allant de 0 à 1999 ; R₁ est un radical monovalent répondant à la formule générale CqH_{2q}L, dans laquelle q est un nombre entier ayant une valeur de 2 à 8 et L est choisi parmi les groupes suivants : -N(R₂)CH₂-CH₂-N(R₂)₂ ; -N(R₂)₂ ; -N(R₂)₃A ; -N(R₂)CH₂-CH₂-NR₂H₂A ; dans laquelle R₂ est un hydrogène, un phényle, un benzyle, ou un radical hydrocarbure saturé ; A est un ion halogénure.

7. Composition de conditionnement des cheveux selon la revendication 7, dans laquelle l'aminosilicone est de formule :
(R₁)ₐG₃₋ₐ-Si-(-OSiG₂)ₙ-(-OSiG_{b}(R₁)_{2-b})ₘ-O-SiG₃₋ₐ(R₁)ₐ
dans laquelle G est un hydrogène, un phényle, un hydroxy, ou un alkyle en C₁ à C₈ ; a est 1 ; n est un nombre de 100 à 1800 ; m est 0 ; R₁ est un radical monovalent répondant à la formule générale CqH_{2q}L, dans laquelle q est un nombre entier ayant une valeur de 2 à 8 et L est -N(R₂)₂ ; dans laquelle R₂ est un hydrogène, un phényle, un benzyle, ou un radical hydrocarbure saturé ; et A est un ion halogénure.

8. Composition de conditionnement des cheveux selon la revendication 8, dans laquelle L est -N(CH₃)₂ ou -NH₂.

9. Composition selon la revendication 1, dans laquelle l'émulsion de copolymère de silicone a une viscosité de phase interne supérieure à 120 x 10⁶ mm²/s, telle que mesurée à 25 °C.

10. Composition selon la revendication 1, dans laquelle l'émulsion de copolymère de silicone comprend un copolymère de silicone, au moins un agent tensioactif, et de l'eau, dans laquelle le copolymère de silicone comprend un polysiloxane ayant des groupes réactifs sur l'une et l'autre terminaisons, et correspondant à la formule suivante : 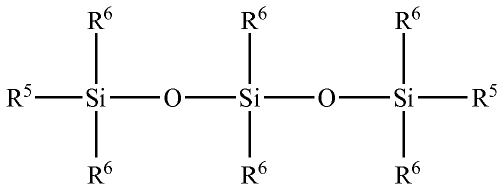 dans laquelle R⁵ est choisi dans le groupe constitué d'un atome d'hydrogène, un groupe aliphatique avec une insaturation éthylénique, un groupe hydroxyle, un groupe alcoxyle, un groupe acétoxyle, un groupe amino ou alkylamino, et leurs mélanges ; et R⁶ est choisi dans le groupe constitué d'un alkyle, un cycloalkyle, un aryle, un alkylaryle, des éthers, des hydroxyles, des amines, des carboxyles, des thiols, des esters, des sulfonates, et leurs mélanges.

11. Composition de conditionnement des cheveux selon la revendication 1, comprenant en outre : de 0,1 % à 10 % en poids de la composition d'un agent tensioactif cationique ; et de 0,1 % à 20 % en poids de la composition d'un composé gras à point de fusion élevé.

12. Composition pour soins capillaires selon la revendication 1, où la composition est un conditionneur à éliminer par rinçage.
